# EUROPÄISCHE PATENTSCHRIFT

(11) **EP 3 590 125 B1**
(45) Veröffentlichungstag und Bekanntmachung des Hinweises auf die Patenterteilung: **04.08.2021**
(21) Anmeldenummer: 18711032.5
(22) Anmeldetag: 02.03.2018
(51) Int. Cl.: H05H 7/08

(54) **VORRICHTUNG ZUM ERZEUGEN BESCHLEUNIGTER ELEKTRONEN**
APPARATUS FOR GENERATING ACCELERATED ELECTRONS
DISPOSITIF POUR PRODUIRE DES ÉLECTRONS ACCÉLÉRÉS

(30) Priorität: 03.03.2017 DE 102017104509
(43) Veröffentlichungstag der Anmeldung: 08.01.2020
(73) Patentinhaber: Fraunhofer-Gesellschaft zur Förderung der angewandten Forschung e.V., 80686 München (DE)
(72) Erfinder: WEIDAUER, André, 01259 Dresden (DE); RÖGNER, Frank-Holm, 01277 Dresden (DE); MATTAUSCH, Gösta, 01454 Ullersdorf (DE); BLÜTHNER, Ralf, 01445 Radebeul (DE); VICENTE GABAS, Ignacio Gabriel, 01309 Dresden (DE); KUBUSCH, Jörg, 01279 Dresden (DE)
(86) Internationale Anmeldenummer: PCT/EP2018/055163
(87) Internationale Veröffentlichungsnummer: WO 2018/158422

(56) Entgegenhaltungen:
- EP-A1- 1 088 797
- EP-A2- 0 231 094
- DE-B3-102013 111 650
- US-A- 3 270 233
- US-A- 3 518 479
- US-A- 3 566 175

## Beschreibung

Die Erfindung betrifft eine Vorrichtung zum Erzeugen beschleunigter Elektronen. Insbesondere kann mit einer erfindungsgemäßen Vorrichtung ein Substrat in einem Substratquerschnitt in einem Behandlungsdurchgang vollumfänglich mit beschleunigten Elektronen beaufschlagt werden. Eine erfindungsgemäße Vorrichtung kann vorteilhaft beim Beaufschlagen von strangförmigen Substraten, Formteilen und Fluiden mit beschleunigten Elektronen eingesetzt werden.

### Stand der Technik

Elektronenstrahltechnologie wird seit etlichen Jahrzehnten im Industriemaßstab zur chemischen Materialmodifikation sowie zur Desinfektion bzw. Sterilisierung von Oberflächen eingesetzt. Die Behandlung von Produkten kann wirtschaftlich vorteilhaft bei atmosphärischem Druck erfolgen, wozu die Elektronen zunächst im Vakuum freigesetzt, anschließend beschleunigt und schließlich durch ein Strahlaustrittsfenster, zumeist eine dünne Metallfolie, in die Behandlungszone ausgekoppelt werden müssen. Zum Durchdringen großtechnisch einsetzbarer, genügend robuster Elektronenaustrittsfenster sowie auch zum Sichern einer ausreichenden Behandlungstiefe im Produkt sind typischerweise Beschleunigungsspannungen >100 kV erforderlich.

Verschiedene Verfahren und Strahlquellen sind für eine Randschichtbehandlung flacher Produkte, wie Platten und Bänder, wohletabliert, während das allseitige Behandeln von Formkörpern, Schüttgütern und Fluiden nach wie vor Probleme bereitet. So ist ein allseitiges gleichmäßiges Beaufschlagen gekrümmter Oberflächen mit Elektronen geometrisch problematisch aufgrund von Abschattungseffekten, variabler Absorption von Elektronenenergie auf der Gasstrecke sowie Dosis-Inhomogenitäten wegen unterschiedlicher Projektionsverhältnisse.

Mit den bereits existierenden Quellensystemen, wie beispielsweise Axialstrahlern mit einer schnellen Ablenkeinheit oder Bandstrahlern mit einer langgestreckten Kathode, von denen beide Ausführungsformen mit einer geheizten thermionischen Kathode betrieben werden, ist eine allseitige Produktbehandlung nur umständlich, unter Nutzung zusätzlicher Einrichtungen oder mit einem hohen apparativen und/oder technologischen Aufwand möglich. Elektronenstrahlquellen auf Basis thermionischer Emitter sind außerdem mechanisch kompliziert, schwierig zu skalieren und erfordern aufwändige Hochspannungsversorgungen und Hochvakuumsysteme. Bei einer Beschädigung des Strahlaustrittsfensters mit daraus resultierendem Zusammenbruch des Vakuums kommt es zur irreversiblen Schädigung des Kathodensystems und somit zu einem hohen Reparaturaufwand.

In DE 199 42 142 A1 ist eine Vorrichtung offenbart, bei der Schüttgut im mehrfachen freien Fall an einer Elektronenstrahleinrichtung vorbeigeführt und mit beschleunigten Elektronen beaufschlagt wird. Aufgrund des Mehrfachdurchlaufs, verbunden mit einer zwischenzeitlichen Durchmischung des Schüttguts, ist die Wahrscheinlichkeit bei dieser Ausführungsform sehr hoch, dass die Partikel des Schüttgutes allseitig mit beschleunigten Elektronen beaufschlagt werden. Der Mehrfachdurchlauf erfordert allerdings einen hohen Zeitaufwand bei der Durchführung des Behandlungsprozesses. Nachteilig ist hierbei außerdem, dass die Vorrichtung für die Behandlung größerer Formteile ungeeignet ist.

Eine andere Lösung ist in DE 10 2006 012 666 A1 angegeben, welche drei Axialstrahler mit zugehöriger Ablenksteuerung und drei ebenfalls zugehörige Elektronenaustrittsfenster umfasst. Die drei Elektronenaustrittsfenster sind derart angeordnet, dass sie einen dreieckigen Freiraum vollumfänglich umschließen. Wird ein Substrat durch diesen Freiraum geführt, kann dieses in einem Behandlungsdurchgang in seinem Querschnitt vollumfänglich mit beschleunigten Elektronen beaufschlagt werden. Hat das Substrat jedoch nicht den gleichen dreieckigen Querschnitt wie der von den drei Elektronenaustrittsfenstern umschlossene Freiraum, wird die Dosisverteilung der Beaufschlagung mit beschleunigten Elektronen auf der Oberfläche des Substrates inhomogen ausfallen. Der apparative Aufwand bei dieser Ausführungsform ist außerdem sehr hoch, wodurch diese Lösung auch sehr preisintensiv ist.

Aus WO 2007/107331 A1 ist eine Vorrichtung bekannt, bei der lediglich zwei Flächenstrahlerzeuger benötigt werden, zwischen denen ein Formteil zum Zwecke des Sterilisierens seiner Oberfläche hindurch bewegt und währenddessen mit beschleunigten Elektronen beaufschlagt werden kann. Diese Vorrichtung weist außerdem mehrere Reflektoren aus Gold auf, mit denen von den Flächenstrahlerzeugern abgegebene Randstrahlen auf Oberflächenbereiche des Formteils reflektiert werden, die nicht im unmittelbaren Einwirkbereich der Flächenstrahlerzeuger liegen. Da die aus dieser Schrift bekannten Reflektoren aus reinem Gold bestehen, sind derartige Vorrichtungen ebenfalls sehr preisintensiv und beeinträchtigen somit deren Wirtschaftlichkeit. Da reflektierte Elektronen eine geringere Energie aufweisen als nicht reflektierte Elektronen, ist auch mit dieser Vorrichtung nur ein inhomogener Energieeintrag in ein Substrat möglich.

Eine ringförmige Vorrichtung zum Erzeugen beschleunigter Elektronen ist in DE 10 2013 111 650 B3 offenbart, bei welcher alle wesentlichen Komponenten, wie beispielsweise Kathode, Anode und Elektronenaustrittsfenster, ringförmig ausgebildet sind, so dass mittels einer solchen Vorrichtung ein ringförmiger Elektronenstrahl ausgebildet werden kann, bei welchem sich die beschleunigten Elektronen zum Ringinneren hin bewegen. Mittels einer solchen Vorrichtung können beispielsweise strangförmige Substrate, die durch die Ringöffnung der Vorrichtung hindurchbewegt werden, bezüglich des Substratquerschnittes vollumfänglich mit beschleunigten Elektronen beaufschlagt werden. Eine aus DE 10 2013 111 650 B3 bekannte Vorrichtung weist üblicherweise eine kreisrunde Ringform auf, kann jedoch auch mit einer beliebig anderen Ringform ausgebildet werden. Bei vielen Anwendungsfällen ist es vorteilhaft, wenn alle Oberflächenbereiche eines Substrates mit möglichst der gleichen Energiedosis beaufschlagt werden. Bei den bekannten Vorrichtungen kann dies realisiert werden, indem beim Herstellen der ringförmigen Strahlungsquelle der Ringquerschnitt an den Strangquerschnitt des zu bestrahlenden strangförmigen Substrates angepasst wird. Nachteilig wirkt sich hierbei aus, dass eine einmal angefertigte ringförmige Strahlungsquelle nur für den Einsatz von Substraten mit einer einzigen Querschnittsform optimal geeignet ist.

### Aufgabenstellung

Der Erfindung liegt daher das technische Problem zugrunde, eine Vorrichtung zum Erzeugen beschleunigter Elektronen zu schaffen, mittels der die Nachteile des Standes der Technik überwunden werden können. Insbesondere sollen mit der Vorrichtung Substrate und insbesondere auch strangförmige Substrate mit verschiedenen Strangquerschnitten vollumfänglich mit beschleunigten Elektronen und oberflächenbezogen, bezüglich des Substrates, mit möglichst gleichmäßiger Elektronendichte beaufschlagt werden können.

Die Lösung des technischen Problems ergibt sich durch Gegenstände mit den Merkmalen des Patentanspruchs 1. Weitere vorteilhafte Ausgestaltungen der Erfindung ergeben sich aus den abhängigen Patentansprüchen.

Ein Merkmal einer erfindungsgemäßen Vorrichtung besteht darin, dass diese ringförmig ausgebildet ist und die Elektronen in Richtung des Ringinneren beschleunigbar sind. Auf diese Weise kann ein Substrat, welches durch das Ringinnere der Vorrichtung geführt wird, in einem Bestrahlungsdurchgang vollumfänglich, bezüglich eines Substratquerschnitts, mit beschleunigten Elektronen beaufschlagt werden. An dieser Stelle sei ausdrücklich darauf verwiesen, dass der Begriff "ringförmig" im Erfindungssinn bei allen nachfolgend beschriebenen ringförmigen Vorrichtungen und Bauelementen nicht nur auf einen Ring in Kreisform begrenzt ist, sondern dass sich der Begriff "ringförmig" im Erfindungssinn lediglich auf einen schleifenförmig in sich geschlossenen Gegenstand bezieht, wobei der schleifenförmig in sich geschlossenen Gegenstand einen Freiraum in seinem Querschnitt vollständig umschließt und wobei ein Substrat durch diesen Freiraum im Inneren des Ringes hindurchgeführt werden kann. Dabei ist der von einem Ring vollständig umschlossene Querschnitt des Freiraumes zwar bei einer bevorzugten Ausführungsform der Erfindung kreisförmig ausgebildet, kann aber im weitesten Erfindungssinn auch jede andere geometrische Form aufweisen.

### Ausführungsbeispiel

Die Erfindung wird nachfolgend anhand eines Ausführungsbeispiels näher erläutert. Die Fig. zeigen:
- Fig. 1: eine schematische und perspektivische Querschnittsdarstellung einer erfindungsgemäßen Vorrichtung;
- Fig. 2: eine schematische Darstellung einer Draufsicht der ringförmigen Vorrichtung aus Fig. 1.

Zum besseren Verständnis der Erfindung seien an dieser Stelle noch die Begriffe "Ringzylinder" und "Ringscheibe" in Bezug auf einen ringförmigen Gegenstand definiert. Wird der Innenradius eines kreisförmigen Ringes von seinem Außenradius subtrahiert, dann ergibt sich ein Maß. Ist dieses Maß kleiner als die Ausdehnung des Ringes in Richtung seiner Ringachse, so ist der Ring als Ringzylinder ausgebildet. Ist dieses Maß hingegen größer als die Ausdehnung des Ringes in Richtung seiner Ringachse, so ist der Ring als Ringscheibe ausgebildet.

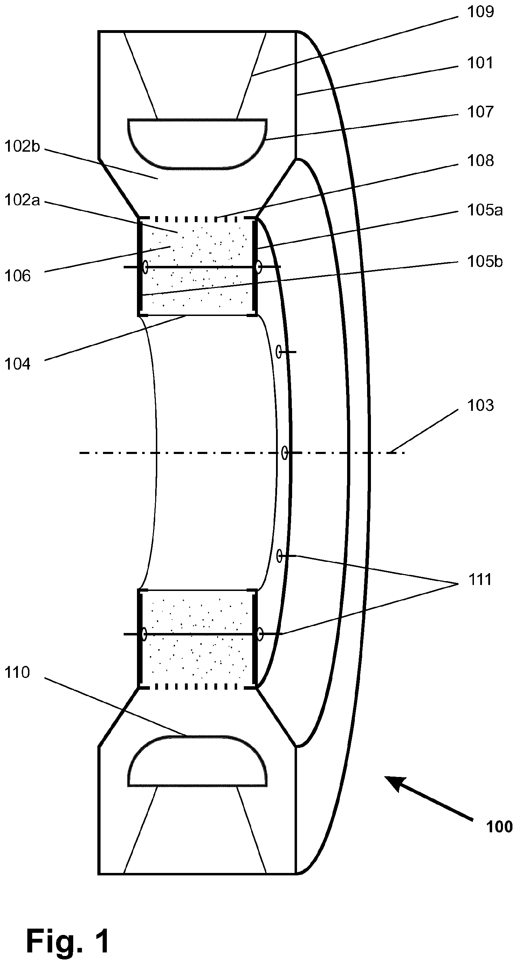

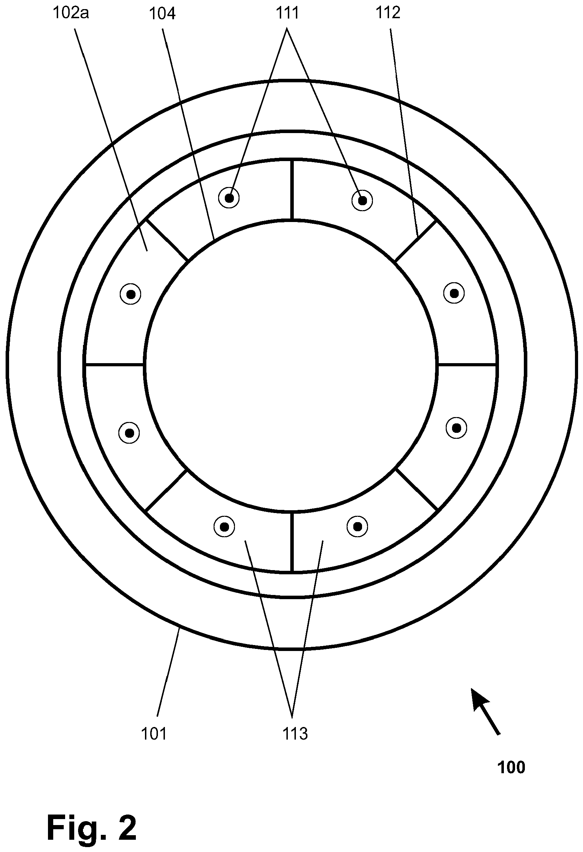

In Fig. 1 und Fig. 2 ist eine identische erfindungsgemäße Vorrichtung 100 schematisch dargestellt, in Fig. 1 als perspektivische Querschnittsdarstellung und in Fig. 2 als Draufsicht.

Eine erfindungsgemäße Vorrichtung umfasst zunächst ein ringförmiges Gehäuse 101, welches zumindest in einem Bereich einen evakuierbaren Raum 102 begrenzt, der in die evakuierbaren Räume 102a und 102b unterteilt ist. Dieser evakuierbare Raum 102 ist aufgrund der Gehäuseform ebenfalls ringförmig. Im Ausführungsbeispiel von Fig. 1 ist das Gehäuse 101 radialsymmetrisch um eine Ringachse 103 ausgebildet. Alle nachfolgend beschriebenen, zu Vorrichtung 100 zugehörigen und als ringförmig bezeichneten Bauelemente sind ebenfalls radialsymmetrisch und weisen ein und dieselbe Ringachse 103 auf. An der Ringinnenseite des Gehäuses 101 ist das Gehäuse 101 als Elektronenaustrittsfenster 104 in Form eines Ringzylinders ausgebildet, d. h. in Austrittsrichtung der Elektronen betrachtet weist das Elektronenaustrittsfenster 104 eine Oberflächensenkrechte auf, die zum Ringinneren und bei einem kreisförmigen Ringzylinder wie beim Elektronenaustrittsfenster 104 zur Ringachse 103 ausgerichtet ist. Durch mindestens einen in Fig. 1 nicht dargestellten Einlass im Gehäuse 101 wird ein Arbeitsgas in den evakuierbaren Raum 102 eingelassen und mittels mindestens einer in Fig. 1 ebenfalls nicht dargestellten Pumpeinrichtung ein Vakuum im evakuierbaren Raum 102 im Bereich von 0,1 Pa bis 20 Pa und bevorzugt im Bereich von 1 Pa bis 3 Pa aufrechterhalten.

Eine erfindungsgemäße Vorrichtung weist ferner mindestens eine erste Kathode und mindestens eine erste Anode auf, zwischen denen mittels einer ersten anlegbaren elektrischen Spannung, die von einer ersten Stromversorgungseinrichtung bereitgestellt wird, ein Glimmentladungsplasma im evakuierbaren Raum 102 erzeugbar ist. Im Ausführungsbeispiel wurden zwei als Ringscheibe geformte Wandungsbereiche des Gehäuses 101 als erste Kathoden 105a und 105b ausgebildet, die im Raum 102a gegenüberliegend begrenzen. Bei der Vorrichtung 100 weisen somit das Gehäuse 101 und die ersten Kathoden 105a, 105b das gleiche elektrische Potenzial auf, welches gleichzeitig das elektrische Massepotenzial der Vorrichtung 100 ist.

Die erste Anode einer erfindungsgemäßen Vorrichtung ist als eine Anzahl drahtförmiger Elektroden ausgebildet, die sich durch den Raum 102a hindurch erstrecken und bei einem Gehäuse in Form eines kreisförmigen Ringes, wie Gehäuse 101, vorzugsweise auf einem identischen Radius und mit gleichem Abstand zueinander um die Achse 103 herum angeordnet sind. Dabei werden die drahtförmigen Elektroden 111, die ein leicht positives Spannungspotenzial in einem Bereich von +0,25 kV bis +5,0 kV gegenüber dem Gehäuse 101 aufweisen können, elektrisch isoliert durch das Gehäuse 101 und die ersten Kathoden 105a, 105b hindurchgeführt. Aufgrund der zwischen den drahtförmigen Elektroden 111 und den ersten Kathoden 105a und 105b angelegten elektrischen Spannung wird ein Plasma im Raum 102a ausgebildet. Der Raum 102a wird deshalb nachfolgend auch als Plasma-Raum 102a bezeichnet.

Eine erfindungsgemäße Vorrichtung umfasst des Weiteren mindestens eine zweite Kathode und mindestens eine zweite Anode, zwischen denen mittels einer zweiten Stromversorgungseinrichtung eine zweite elektrische Spannung geschaltet ist. Bei Vorrichtung 100 ist eine ringförmige Kathode 107 als zweite Kathode und eine ringförmige und gleichzeitig gitterförmige Anode 108 als zweite Anode ausgebildet.

Die zweite Kathode stellt bei einer erfindungsgemäßen Vorrichtung die Kathode zum Emittieren von Sekundärelektronen dar, welche anschließend beschleunigt werden, und weist hierfür ein elektrisches Hochspannungspotenzial, bevorzugt im Bereich von -100 kV bis -300 kV, auf. Mittels eines Isolators 109 ist die zweite Kathode 107 elektrisch gegenüber dem Gehäuse 101 isoliert.

Bei der in Fig. 1 beschriebenen Ausführungsform der Erfindung weisen die zweite Anode 108 und die ersten Kathoden 105a, 105b das gleiche elektrische Potenzial auf, welches als elektrisches Massepotenzial ausgebildet ist. Alternativ können die zweite Anode und die erste Kathode auch unterschiedliche elektrische Potenziale aufweisen.

Aus dem Plasma 106 im Raum 102a werden durch das Anlegen eines Hochspannungspotenzials im Bereich von -100 kV bis -300 kV Ionen durch die gitterförmige zweite Anode 108 in Richtung der zweiten Kathode 107 beschleunigt. Dort treffen die Ionen auf einen Oberflächenbereich 110 der zweiten Kathode 107, dessen Oberflächensenkrechte zum Ringinneren des Gehäuses und bei einem radialsymmetrischen Gehäuse, wie Gehäuse 101, zur Ringachse 103 ausgerichtet ist. Beim Auftreffen der Ionen auf den Oberflächenbereich 110 haben die Ionen somit eine Potenzialdifferenz durchfallen, die weitgehend der Beschleunigungsspannung der Vorrichtung 100 entspricht. Bei ihrem Auftreffen wird die Energie der Ionen in einer sehr dünnen Randschicht der Kathode 107 im Oberflächenbereich 110 frei, was zur Auslösung von Sekundärelektronen führt. Bei den zuvor genannten elektrischen Spannungen an der zweiten Kathode 107 ist das Verhältnis zwischen ausgelösten Elektronen und auftreffenden Ionen in der Größenordnung 10 angesiedelt, was diese Art des Erzeugens beschleunigter Elektronen sehr effizient macht. Die entstandenen Sekundärelektronen werden vom anliegenden elektrischen Feld stark beschleunigt und durchfliegen die in Form eines Ringzylinders ausgebildete gitterförmige Anode 108 und das Plasma 106 im Raum 102a. Nach dem Durchqueren des Elektronenaustrittsfensters 104, das beispielsweise als dünne Metallfolie ausgeführt sein kann, dringen die Elektronen in den vom ringförmigen Gehäuse 101 umschlossenen Freiraum vor, in dem ein höherer Druck als im Raum 102 herrschen kann und durch den ein mit Elektronen zu beaufschlagendes Substrat durch die Gehäuseringöffnung hindurchgeführt werden kann. Als Material für das Elektronenaustrittsfenster 104 können alle aus dem Stand der Technik für ein Elektronenaustrittsfenster bekannten Materialien, wie beispielsweise Titan, verwendet werden. Außerdem ist es zum Zwecke einer höheren mechanischen Stabilität des Elektronenaustrittsfensters 104 vorteilhaft, dieses mit einem Stützgitter zu versehen, wie es ebenfalls aus dem Stand der Technik bekannt ist.

Aufgrund der ringförmigen Gestalt aller zuvor genannten Bauteile einer erfindungsgemäßen Vorrichtung wird mit dieser ein in sich geschlossenes, ringförmiges Band beschleunigter Elektronen erzeugt, wobei die Bewegungsrichtung der beschleunigten Elektronen auf den vom Gehäusering eingeschlossenen Freiraum ausgerichtet ist. Der Freiraum, der vom Gehäusering umschlossen wird und durch den ein Substrat hindurchgeführt werden kann, wird nachfolgend auch als Behandlungsraum bezeichnet. Bei einer radialsymmetrischen erfindungsgemäßen Vorrichtung, wie Vorrichtung 100, ist die Bewegungsrichtung der beschleunigten Elektronen vorzugsweise auf die Ringachse 103 ausgerichtet. Ein Substrat, welches durch das Ringinnere des Gehäuses einer erfindungsgemäßen Vorrichtung geführt wird, kann somit vollumfänglich, bezüglich eines Substratquerschnitts, in einem Durchlauf mit beschleunigten Elektronen beaufschlagt werden. Eine erfindungsgemäße Vorrichtung ist daher besonders für das Beaufschlagen mit beschleunigten Elektronen von strangförmigen Substraten, von Formteilen aber auch von Fluiden geeignet.

Der Vollständigkeit halber sein an dieser Stelle erwähnt, dass eine erfindungsgemäße Vorrichtung auch eine Einrichtung zum Kühlen der Vorrichtung aufweist, wie es auch bei Vorrichtungen zum Erzeugen beschleunigter Elektronen aus dem Stand der Technik bekannt ist. So kann diese Einrichtung zum Kühlen einer erfindungsgemäßen Vorrichtung beispielsweise Kühlkanäle umfassen, die sich innerhalb des Isolators 109 erstrecken und durch die ein Kühlmedium strömt.

Die zweite Anode 108, welche bei einer erfindungsgemäßen Vorrichtung bevorzugt als gitterförmiger Ringzylinder ausgebildet ist und welche die räumliche Grenze zwischen den evakuierbaren Räumen 102a und 102b darstellt, erfüllt drei wesentliche Aufgaben. Zum einen bewirkt sie aufgrund ihrer Spannungsdifferenz gegenüber der zweiten Kathode 107 eine Beschleunigung der aus dem Plasma extrahierten Ionen in Richtung der zweiten Kathode. Zum anderen bewirkt sie auch eine Beschleunigung der durch den lonenbeschuss erzeugten Sekundärelektronen in Richtung des Elektronenaustrittsfensters 104. Aufgrund des Sachverhaltes, dass die ringförmige Gitterstruktur der zweiten Anode 108 parallel zur Sekundärelektronen emittierenden Oberfläche 110 der zweiten Kathode 107 ausgebildet ist, wird ein elektrisches Feld derart ausgebildet, dass auch die Bahnen der beschleunigten Sekundärelektronen weitgehend parallel verlaufen. Des Weiteren schirmt die zweite Anode 108 das Plasma vom Spannungspotenzial der zweiten Kathode 107 ab; verhindert dadurch das Abdriften zu vieler Ionen in Richtung zweiter Kathode 107 und trägt somit zum Aufrechterhalten des Plasmas 106 im Raum 102a bei.

Bei der erfindungsgemäßen Vorrichtung ist der ringförmige Plasma-Raum 102a mittels Wandungen 112 in Ringsegmente 113 unterteilt, wie aus Fig. 2 ersichtlich ist. Bei der Vorrichtung 100 bestehen die Wandungen 112 aus einem elektrisch leitfähigen Material und weisen das gleiche elektrische Spannungspotenzial auf wie das Gehäuse 101, die ersten Kathoden 105a, 105b und auch das Elektronenaustrittsfenster 104. Im Ausführungsbeispiel ist dieses elektrische Spannungspotenzial das elektrische Massepotenzial. Dabei weist jedes Ringsegment 113 mindestens eine drahtförmige Elektrode 111 auf, die sich durch das Ringsegment 113 vorzugsweise parallel zur Ringachse 103 erstreckt. Ferner ist jedem Ringsegment 113 eine in den Figuren nicht dargestellte separate Stromversorgungseinrichtung zugeordnet, mittels welcher die Stärke des elektrischen Stromes, der durch die mindestens eine Elektrode 111 eines jeweiligen Ringsegments 113 fließt, einstellbar ist.

Auf Grund der zuvor beschriebenen Spannungspotenzialverhältnisse fungieren bei Vorrichtung 100 die ersten Kathoden 105a, 105b, die zugehörigen Wandungen 112 als auch ein jeweiliger zugehöriger Abschnitt des Elektronenaustrittsfensters 104 als Kathode für die Plasmaentladung innerhalb eines jeweiligen Ringsegments 113.

Das Unterteilen des ringförmigen Plasma-Raums 102a in Ringsegmente 113 im Zusammenspiel mit dem separaten Steuern der Stärke des Stromes, der durch die mindestens eine drahtförmige Elektrode 111 eines Ringsegments 113 fließt, ermöglichen es, dass innerhalb jedes Ringsegments 113 ein separates Plasma mit einer separaten Plasmastärke ausgebildet werden kann. Die Menge an Elektronen, die über den einem jeweiligen Ringsegment 113 zugehörigen Flächenabschnitt des Elektronenaustrittsfensters 104 abgestrahlt werden, ist somit für jedes Ringsegment 113 separat einstellbar. Folglich kann das ringförmige Emissionsprofil einer erfindungsgemäßen Vorrichtung an die Kontur der Querschnittsfläche bzw. an den unterschiedlichen Dosisbedarf einzelner Oberflächenbereiche eines mit beschleunigten Elektronen zu beaufschlagenden Substrates angepasst werden. Sind die Wandungen 112 einer erfindungsgemäßen Vorrichtung mittels einer rückbaubaren Montagevariante in der Vorrichtung integriert, lässt sich durch das Versetzen von Wandungen 112 und/oder das Verändern der Anzahl der verwendeten Wandungen 112 im Zusammenspiel mit dem veränderten Ansteuern von drahtförmigen Elektroden, die dann zu einem Ringsegment gehören, eine weitere Anpassung an Substratprofile erzielen ohne gleich eine gänzlich neue Strahlvorrichtung herstellen zu müssen.

Bei der in den Fig. 1 und 2 dargestellten Vorrichtung 100 ist der ringförmige Plasma-Raum 102a lediglich beispielhaft in 8 gleich große Ringsegmente 113 unterteilt. Alternativ kann der Plasma-Raum 102a einer erfindungsgemäßen Vorrichtung auch in eine beliebig andere Anzahl von Ringsegmenten unterteilt werden, die bei einer Ausführungsform auch eine unterschiedliche Größe, bezogen auf den Ringwinkel, aufweisen können. Auch ist die Anzahl von einer drahtförmigen Elektrode 111 pro Ringsegment 113 nur beispielhaft gewählt. Alternativ können die Ringsegmente einer erfindungsgemäßen Vorrichtung auch mehr als nur eine drahtförmige Elektrode aufweisen.

## Patentansprüche

1. Vorrichtung zum Erzeugen beschleunigter Elektronen, umfassend ein Gehäuse (101), welches einen evakuierbaren Raum (102a; 102b) begrenzt und ein Elektronenaustrittsfenster (104) aufweist; einen Einlass für das Zuführen eines Arbeitsgases in den evakuierbaren Raum (102); mindestens eine erste Kathode (105a, 105b) und mindestens eine erste Anode, zwischen denen mittels einer ersten anlegbaren elektrischen Spannung ein Glimmentladungsplasma (106) im evakuierbaren Raum (102a) erzeugbar ist, wobei Ionen aus dem Glimmentladungsplasma (106) auf die Oberfläche (110) einer zweiten Kathode (107) beschleunigbar sind und von der zweiten Kathode (107) emittierbare Elektronen mittels einer zwischen der zweiten Kathode (107) und einer zweiten Anode (108) anlegbaren zweiten elektrischen Spannung in Richtung Elektronenaustrittsfenster (104) beschleunigbar sind, wobei das Gehäuse (101), die zweite Kathode (107) und das Elektronenaustrittsfenster (104) ringförmig ausgebildet sind, wobei die Oberflächensenkrechten des Elektronenaustrittsfensters (104) und des Oberflächenbereichs (110) der zweiten Kathode (107), aus dem Elektronen emittierbar sind, zum Ringinneren des ringförmigen Gehäuses (101) ausgerichtet sind, wobei die erste Anode als eine Anzahl drahtförmiger Elektroden (111) ausgebildet ist, die sich durch den Raum (102a) hindurch erstrecken, **dadurch gekennzeichnet,**
**dass** der ringförmige Raum (102a) mittels Wandungen (112) in Ringsegmente (113) unterteilt ist, wobei jedes Ringsegment (113) mindestens eine drahtförmige Elektrode (111) aufweist, die sich durch das Ringsegment (113) hindurch erstreckt und jedem Ringsegment (113) mindestens eine separate Stromversorgungseinrichtung zugeordnet ist, mittels der die Stärke des elektrischen Stromes, der durch die mindestens eine Elektrode (213) eines jeweiligen Ringsegments fließt, einstellbar ist.

2. Vorrichtung nach Anspruch 1, **dadurch gekennzeichnet, dass** die Wandungen (112) aus einem elektrisch leitfähigen Material bestehen.

3. Vorrichtung nach Anspruch 2, **dadurch gekennzeichnet, dass** die Wandungen (112) das gleiche elektrische Potenzial aufweisen wie das Gehäuse (101).

4. Vorrichtung nach Anspruch 3, **dadurch gekennzeichnet, dass** die Wandungen (112) elektrisches Massepotenzial aufweisen.

5. Vorrichtung nach Anspruch 1, **dadurch gekennzeichnet, dass** das Gehäuse (101) und die erste Kathode (105a; 105b) das gleiche elektrische Potenzial aufweisen.

6. Vorrichtung nach Anspruch 1, **dadurch gekennzeichnet, dass** das Gehäuse (101) und die zweite Anode (108) das gleiche elektrische Spannungspotenzial aufweisen.

7. Vorrichtung nach Anspruch 1, **dadurch gekennzeichnet, dass** die zweite Anode (108) als gitterförmiger Ringzylinder ausgebildet ist.

8. Vorrichtung nach Anspruch 1, **dadurch gekennzeichnet, dass** die drahtförmigen Elektroden (111) auf einem identischen Radius und mit gleichem Abstand zueinander um die Ringachse (103) des Gehäuses (101) herum angeordnet sind.

## Claims

1. Apparatus for generating accelerated electrons, comprising a housing (101), which delimits an evacuable space (102a; 102b) and comprises an electron exit window (104); an inlet for the supply of a working gas into the evacuable space (102); at least one first cathode (105a, 105b) and at least one first anode, between which a glow discharge plasma (106) is able to be generated in the evacuable space (102a) by means of a first applicable voltage, wherein ions from the glow discharge plasma (106) are able to be accelerated towards the surface (110) of a second cathode (107) and electrons which are able to be emitted by the second cathode (107) are able to be accelerated in the direction of the electron exit window (104) by means of a second voltage applicable between the second cathode (107) and a second anode (108), wherein the housing (101), the second cathode (107), and the electron exit window (104) have a ring-shaped form, wherein the surface perpendicular lines of the electron exit window (104) and of the surface region (110) of the second cathode (107), from which electrons are able to be emitted, are oriented towards the ring interior of the ring-shaped housing (101), wherein the first anode is formed as a number of wire-shaped electrodes (111), which extend through the space (102a), **characterized in that** the ring-shaped space (102a) is divided by means of walls (112) into ring segments (113), wherein each ring segment (113) comprises at least one wire-shaped electrode (111), which extends through the ring segment (113), and at least one separate power supply unit is assigned to each ring segment (113), by means of which the strength of the electric current which flows through the at least one electrode (213) of a respective ring segment is adjustable.

2. Apparatus according to Claim 1, **characterized in that** the walls (112) consist of an electrically conductive material.

3. Apparatus according to Claim 2, **characterized in that** the walls (112) have the same electric potential as the housing (101).

4. Apparatus according to Claim 3, **characterized in that** the walls (112) have electric earth potential.

5. Apparatus according to Claim 1, **characterized in that** the housing (101) and the first cathode (105a; 105b) have the same electric potential.

6. Apparatus according to Claim 1, **characterized in that** the housing (101) and the second anode (108) have the same electric potential.

7. Apparatus according to Claim 1, **characterized in that** the second anode (108) is formed as a grid-shaped ring cylinder.

8. Apparatus according to Claim 1, **characterized in that** the wire-shaped electrodes (111) are arranged at an identical radius and with the same spacing from one another around the ring axis (103) of the housing (101).

## Revendications

1. Dispositif de génération d'électrons accélérés, ledit dispositif comprenant un boîtier (101) qui délimite un espace évacuable (102a ; 102b) et qui comporte une fenêtre de sortie d'électrons (104) ; une entrée destinée à amener un gaz de travail dans l'espace évacuable (102) ; au moins une première cathode (105a, 105b) et au moins une première anode, entre lesquelles un plasma de décharge luminescente (106) peut être généré dans l'espace évacuable (102a) au moyen d'une première tension électrique pouvant être appliquée, des ions provenant du plasma de décharge luminescente (106) pouvant être accélérés jusque sur la surface (110) d'une deuxième cathode (107) et des électrons qui peuvent être émis par la deuxième cathode (107) pouvant être accélérés en direction de la fenêtre de sortie d'électrons (104) au moyen d'une deuxième tension électrique qui peut être appliquée entre la deuxième cathode (107) et une deuxième anode (108), le boîtier (101), la deuxième cathode (107) et la fenêtre de sortie d'électrons (104) étant de forme annulaire, les perpendiculaires de surface de la fenêtre de sortie d'électrons (104) et de la zone de surface (110) de la deuxième cathode (107), à partir de laquelle des électrons peuvent être émis, étant orientées vers l'intérieur annulaire du boîtier annulaire (101), la première anode étant conçue comme un certain nombre d'électrodes filiformes (111) qui s'étendent à travers l'espace (102a),
**caractérisé en ce que** l'espace annulaire (102a) est divisé en segments annulaires (113) au moyen de parois (112), chaque segment annulaire (113) comportant au moins une électrode filiforme (111) qui s'étend à travers le segment annulaire (113) et chaque segment annulaire (113) étant associé à au moins un moyen d'alimentation en courant séparé permettant de régler l'intensité du courant électrique qui circule à travers l'au moins une électrode (213) d'un segment annulaire respectif.

2. Dispositif selon la revendication 1, **caractérisé en ce que** les parois (112) sont en un matériau électriquement conducteur.

3. Dispositif selon la revendication 2, **caractérisé en ce que** les parois (112) sont au même potentiel électrique que le boîtier (101).

4. Dispositif selon la revendication 3, **caractérisé en ce que** les parois (112) sont à un potentiel de masse électrique.

5. Dispositif selon la revendication 1, **caractérisé en ce que** le boîtier (101) et la première cathode (105a ; 105b) sont au même potentiel électrique.

6. Dispositif selon la revendication 1, **caractérisé en ce que** le boîtier (101) et la deuxième anode (108) sont au même potentiel de tension électrique.

7. Dispositif selon la revendication 1, **caractérisé en ce que** la deuxième anode (108) est réalisée sous la forme d'un cylindre annulaire en forme de treillis.

8. Dispositif selon la revendication 1, **caractérisé en ce que** les électrodes filiformes (111) sont disposées sur un même rayon et à une même distance les unes des autres autour de l'axe annulaire (103) du boîtier (101).
